(19) **Europäisches Patentamt**

**European Patent Office**

**Office européen des brevets**

(11) Veröffentlichungsnummer : **0 343 694 B1**

(12) # EUROPÄISCHE PATENTSCHRIFT

(45) Veröffentlichungstag der Patentschrift :
**25.11.92 Patentblatt 92/48**

(21) Anmeldenummer : **89112798.7**

(22) Anmeldetag : **28.02.85**

(51) Int. Cl.$^5$ : **A61K 35/78, A61K 31/355**

---

(54) **Mittel zur Behandlung und zum Schutz der Haut.**

---

Die Akte enthält technische Angaben, die nach dem Eingang der Anmeldung eingereicht wurden und die nicht in dieser Patentschrift enthalten sind.

(30) Priorität : **07.03.84 DE 3408258**
**23.03.84 DE 3410641**
**01.06.84 DE 3420459**
**25.07.84 DE 3427374**
**25.09.84 DE 3435098**
**15.11.84 DE 3441711**
**12.02.85 DE 3504695**

(43) Veröffentlichungstag der Anmeldung :
**29.11.89 Patentblatt 89/48**

(45) Bekanntmachung des Hinweises auf die Patenterteilung :
**25.11.92 Patentblatt 92/48**

(84) Benannte Vertragsstaaten :
**AT BE CH DE FR GB IT LI LU NL SE**

(56) Entgegenhaltungen :
**US-A- 4 248 861**
**UNLISTED DRUGS, Band 28, Nr. 7, Juli 1976, (CHATHAM, New Jersey, US)**

(60) Veröffentlichungsnummer der früheren Anmeldung nach Art. 76 EPÜ : **0 158 090**

(73) Patentinhaber : **Ismail, Roshdy, Dr.**
**Siebengebirgs-Apotheke Siebengebirgsallee 2**
**W-5000 Köln 41 (Klettenberg) (DE)**

(72) Erfinder : **Ismail, Roshdy, Dr.**
**Siebengebirgs-Apotheke Siebengebirgsallee 2**
**W-5000 Köln 41 (Klettenberg) (DE)**

(74) Vertreter : **Werner, Hans-Karsten, Dr. et al**
**Deichmannhaus am Hauptbahnhof**
**W-5000 Köln 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Gegenstand der vorliegenden Erfindung ist die Verwendung einer Zusammensetzung zur Behandlung und zum Schutz der Haut unter Einsatz von Vitamin E nach den Ansprüchen 1 bis 5.

Vitamin E ist bekannt als Antioxidans und Schutzvitamin für Phosphorlipide der Zellmembran. Es hält die Permeabilität und Stabilität der Zellmembran aufrecht; Lucy, Ann. N.Y. Academy of Science 203 1972, S. 4. Es ist weiterhin bekannt, daß Vitamin E eine membranabdichtende Wirkung besitzt; F. Mittelbach und G. Bodechtel, Münchner Medizinische Wochenschrift 110 (1968) 36: 1988-1993. Bei Erythrocyten, den einfachsten Zellen des menschlichen Körpers wurde festgestellt, daß Vitamin E eine Schutzwirkung für die Zellmembran darstellt. In Tierversuchen und beim Menschen wurde bewiesen, daß Anämie das erste Anzeichen von Vitamin-E-Mangel ist. Bei Gabe von hohen Vitamin-E-Dosen normalisiert sich die Hämolyse der Erythrocyten; vgl. William J. Darbey Vitamin Horm, 26 (50) S. 685-704 (1968) und Phelps DL Pediatrics 63 (6) S. 933-935 (1979). Aus diesen Literaturstellen geht hervor, daß bei oraler Verabreichung von 200 bis 800 mg Vitamin E an Patienten für einen Zeitraum von 1 bis 4 Tagen, deren Hämolyse der Erythrocyten significant verbessert wird im Vergleich zu Patienten mit Vitamin-E-Mangel.

Vitamin E ist weiterhin verwendet worden zur Behandlung der Sichelzellenanämie in einem Zeitraum von 6 bis 35 Wochen; vgl. Natt CL. Am. J. clin. 33, S. 968-971 (1980); Natt CL. Am. J. clin. nutr. 32, S. 1359-1362 (1979); Gawlik G.M. Fed. Proc. 35 (3), S. 252 (1976) und Gorash L. Bieri J.G. et al univ. Conn. Farmington, GT.

Weiterhin ist bekannt, daß 750 mg Vitamin E täglich in einem Zeitraum von 3 bis 6 Monaten erfolgreich bei Thalassamie-Patienten eingesetzt wurden, wobei eine Normalisierung der Hämolyse der Erythrocyten beobachtet wurde; vgl. Kahane I. ISR. J. med. 12 (1), S. 11-15 (1976).

Erfolgreich eingesetzt wurde Vitamin E weiterhin bei Patienten mit akuter Hepatitis und alkoholischer Hepatitis, die einen Mangel an Vitamin E im Serum haben; vgl. Yoshiakawa T. Takemura S. Kato H. et al. Japan J. Gastrovent, 74/7, S. 732-739 (1977). Schließlich wurde Vitamin E bei Patienten mit Eisenmangelanämie eingesetzt und bewirkte während eines Zeitraumes von 4 bis 8 Wochen eine Verbesserung bzw. Normalisierung des Lipidmetabolismus im Knochenmark; vgl. Takoshi Itaga, Central Clinical Laboratory Nagasaki University of Medicine, Japan.

In "Unlisted Durgs", Band 28, Nr. 7, 1976, 119n (New Jersey), wird eine Formulierung enthaltend d-$\alpha$-Tocopherol-acetat, Troxerutin und Vincamin zur Behandlung von Funktionsstörungen des Kreislaufs beschrieben.

In den deutschen Patentanmeldungen P 34 20 738, P 34 05 928, P 34 05 239, P 34 07 025, P 34 08 260, P 34 16 162, P 34 32 881, P 34 05 240, P 34 02 930, P 34 07 024, P 34 07 026, P 34 15 250, P 34 27 193 wird ferner der Einsatz von Vitamin E zur Behandlung der Venen, des Analbereichs und von Rheumaerkrankungen vorgeschlagen.

Es ist weiterhin bekannt, daß Cholesterin in menschlicher und tierischer Haut durch Ultraviolett-Licht zu Cholesterin-alpha-oxyd, einen als Krebserreger bekannten Stoff, umgewandelt wird. Versuche mit Mäusen haben gezeigt, daß bei Verabreichung von Vitamin E und C sowie zwei weiteren Antioxidantien sich kein Cholesterin-alpha-oxyd bildet (Pharm. Indu. 36, Nr. 3 (1974) Anschel, USA).

Die US-A-4,248,861 betrifft ein Verfahren zur Prävention schädlicher Effekte von Sonnenstrahlung auf menschlicher Haut. Die dort offenbarten Zubereitungen werden vor allem zur Behandlung von bereits verbrannter Haut eingesetzt. Sie enthalten neben 5 bis 20 Teilen Para-Aminobenzoesäure, 1 bis 10 Teile Calcium-d-Pantothenat und 0,5 bis 1,5 Teile einer Tocopherol-Verbindung. Diese Zubereitungen sollen auch vorbeugend eine gewisse Wirksamkeit haben. Von diesen Zubereitungen wird angenommen, daß sie das Vitamingleichgewicht in der Hautzellstruktur wiederherstellen. Die Menge an Tocopherol-Verbindungen liegt in diesen Zubereitungen zwischen 150 und 500 IE pro F1 ounce entsprechend 0,5 bis 1,8 Gew.-%. Eine unmittelbare Schutzwirkung gegen schädliche Strahlung wird aber nicht offenbart.

Es wurde nun überraschenderweise gefunden, daß die Verwendung einer Zusammensetzung, die Vitamin-E sowie Kombinationen von Vitamin E mit anderen Wirkstoffen neben üblichen Träger- und/oder Hilfsstoffen, sich insbesondere zur Behandlung von Ekzemen, Hautflechte, Hautentzündungen, Juckreiz, Allergien, Faltenbildungen, Pigmentierungen der Haut und Haarausfall sowie Wunden, eignen. Darüberhinaus können die erfindungsgemäß verwendbaren Zusammenstellungen als Schutz gegen ultra-violettes Licht und zur Förderung des Haarwuchses eingesetzt werden. Erfindungsgemäß kann die Zusammensetzung ferner als Hautschutzmittel bei Bestrahlungen, z.B. von Krebspatienten, verwendet werden. Dieser neue Indikationsbereich war aufgrund des bisherigen Wissensstandes nicht vorherzusehen und eröffnet ein neues breites Anwendungsfeld für Vitamin E. Die Verwendung von Vitamin E bringt auf lange Sicht eine Stabilisierung und dauernde Beseitigung der Symptome, die Wahrscheinlichkeit der Rückfälligkeit ist dadurch sehr gering. Die Vitamin-E-haltigen Kombinationspräparate müssen jedoch längere Zeit, ca. 6 Monate oder länger, angewendet werden.

EP 0 343 694 B1

Gegenstand der vorliegenden Erfindung ist die Verwendung einer Zusammensetzung, die neben üblichen Träger- und/oder Hilfsstoffen als wesentlichen Bestandteil Vitamin E als alpha-Tocopherol und/oder dessen Ester natürlicher oder synthetischer Herkunft in Mengen von 200 bis 1000 mg pro Darreichungseinheit sowie durchblutungsfördernde und/oder gefäßerweiternde Mittel und außerdem gegebenenfalls Vitamin C, Vitamin A, Vitamine der B-Reihe, Phospholipide, ungesättigte Fettsäuren und/oder Emulgatoren enthält, zur Herstellung eines Arzneimittels in Kapselform zur Behandlung und zum Schutz der menschlichen und tierischen Haut; Verwendung einer Zusammensetzung, die neben üblichen Träger- und/oder Hilfsstoffen als wesentlichen Bestandteil Vitamin E als alpha-Tocopherol und/oder dessen Ester natürlicher oder synthetischer Herkunft in Mengen von 0,5 bis 20 Gew.-% pro Darreichungseinheit sowie durchblutungsfördernde und/oder gefäßerweiternde Mittel und außerdem gegebenenfalls Vitamin C, Vitamin A, Vitamine der B-Reihe, Phospholipide, ungesättigte Fettsäuren und/oder Emulgatoren enthält, zur Herstellung eines Arzneimittels in Form von Crême, Gel, Salbe, Milch Lotion oder Lösung zur Behandlung und zum Schutz der menschlichen und tierischen Haut sowie die

Verwendung einer Zusammensetzung, die neben üblichen Träger- und/oder Hilfsstoffen als wesentlichen Bestandteil Vitamin E als alpha-Tocopherol und/oder dessen Ester natürlicher oder synthetischer Herkunft in Mengen bis zu 32 Gew.-% pro Darreichungseinheit sowie durchblutungsfördernde und/oder gefäßerweiternde Mittel, und außerdem gegebenenfalls Vitamin C, Vitamin A, Vitamine der B-Reihe, Phospholipide, ungesättigte Fettsäuren und/oder Emulgatoren enthält, zur Herstellung eines Arzneimittels in Form von Spray, Tinktur oder Lösung in Alkohol zur Behandlung und zum Schutz der menschlichen und tierischen Haut.

Da Vitamin E bei Raumtemperatur flüssig ist, bietet sich als Applikationsform insbesondere die Kapsel an. Die übrigen Wirkstoffe werden in Vitamin E sowie gewünschtenfalls in einem dünnflüssigen Neutralöl und einem Lösungsvermittler in an sich bekannter Weise in die Kapsel eingebracht. Auch hier können Emulgatoren, z.B. Tween, eingesetzt werden.

Bei der erfindungsgemäß zu verwendenden Zusammensetzung ist für die Wirksamkeit von Vitamin E vor allem eine ausreichende Dosierung entscheidend, die mindestens 200 mg betragen sollte. Niedrigere Dosierungen an Vitamin E sind wenig sinnvoll, da erhebliche Mengen durch die Magensäure zerstört werden und dadurch ihre Wirksamkeit verlieren (vgl. Arthur Vogelsang, Angiology 21, S. 275 bis 279 (1970)).

Sofern in der Vergangenheit hin und wieder geringe Mengen Vitamin E, nämlich bis zu 40 mg in Kombinationspräparaten zum Einsatz gekommen sind, waren sie mit Sicherheit wegen der zu niedrigen Dosierung völlig wirkungslos. Zur Behandlung sollte die Dosierung im Bereich von 200 bis 1.000 mg liegen. Vorzugsweise werden Darreichungsformen, die 250 bis 600 mg enthalten, eingesetzt. Typische Kombinationspräparate enthalten 300 bis 500 mg Vitamin E, jeweils pro Darreichungseinheit.

Vitamin E kann in allen seinen alpha-Formen verwendet werden, sowohl als freies Tocopherol als auch als Ester natürlicher oder synthetischer Herkunft. Dieser Ester kann als Acetat, Succinat oder als anderer Ester verwendet werden. Für Salben, Gele und Cremes wird bevorzugt das freie Tocopherol, z.B. D,L-alpha-Tocopherol und D-alpha-Tocopherol verwendet.

Überraschenderweise wird die Wirkung von Vitamin E in Gegenwart von gefäßerweiternden und/oder durchblutungsfördernden Mitteln erheblich gesteigert in Form von Synergismen und dadurch die Behandlungszeit verkürzt.

Insbesondere in Gegenwart der durchblutungsfördernden Mitteln, wie Heparin Natrium oder Extr. Hippocastani wird die Aufnahme des Vitamin E durch die Haut verbessert. Bei Verwendung von Heparin Natrium wird die hohe Dosierung von 30.000 bis 150.000 I.E. bevorzugt.

Weitere verwendbare Zubereitungen im Sinne der Erfindung, die die Wirkung von Vitamin E erheblich steigern, sind durchblutungsfördernde Mittel, wie β-Hydroxy-äthyl-rutosid, Trimethylolrutosid, Arnicae-Extract, Nicotinsäure, Nicotinsäureester und Derivate, Xantinolnicotinat und Inositolnicotinat, sowie Salicylsäure bzw. deren Ester, Dihydroergotoxin-methan-sulphonat, Dihydroergocornin-methan-sulphonat, Dihydroergocristin-methan-sulphonat, β-Hydroxy-äthyl-salicylat, Ol. Juniperi, Ol. Pini pumilionis (Latschenkiefernöl), Ol. Eucalypti, Ol. Rosmarinae, Tinct. Camphorae bzw. Kampfer, Cinnarizin, Vincamin, Pentoxyfyllin, Bamethansulfat, Bencyclanhydrogenfumarat, β-Pyridilcarbinol, Ginkgoflavonglykoside.Es können auch weitere Derivate der durchblutungs- bzw. gefäßerweiternden Mittel verwendet werden.

Als gefäßerweiterndes Pflanzenmittel ist Extract Calendulae aus Herba Calendulae zu nennen. Die durchblutungsfördernden Mittel können ebenfalls in Retard-Form verwendet werden. Zahlreiche durchblutungsfördernde Mittel, wie Hydroxy-rutoside haben auch anticoagulierende Eingenschaften.

Die erfindungsgemäß verwendbaren Zusammensetzungen zur Herstellung eines Arzneimittels zur Behandlung und zum Schutz der Haut können auch äußerlich in Form von Creme, Gel, Salbe oder Lotion oder Lösung ggf. zusammen mit Emulgatoren angewendet werden. Die Vitamin E-Konzentration beträgt in diesem Fall 0,5 bis 20 Gew.-%. Besonders bevorzugt werden 4 bis 10 Gew.-%. Man kann auch andere Darreichungsformen zubereiten, z.B. Sprays, Tinkturen oder alkoholische Lösungen. Isopropanol bzw. Propandiol ist ein be-

3

sonders bevorzugtes Lösungsmittel, das zugleich durchblutungsfördernd wirkt. Die Konzentration des Vitamin E kann in diesem Fall 32 Gew.-% betragen. Bevorzugt wird eine Konzentration bis zu 25 Gew.-% pro Darreichungsform.

Als übliche Salben oder Cremegrundlagen können Eucerin cum. aqua, Ungt. Cordes, Ungt. Emulsificans, sowie andere nicht wasserlösliche Salbengrundlagen bzw. deren Gemische verwendet werden. Geeignete Salbengrundlagen sind beispielsweise Wollwachs, Vaseline DAB 8, Paraffin dünnflüssig sowie Gemische derselben. Sie können auch Emulgatoren enthalten wie Cetylstearylalkohol. Geeignet als Salbengrundlagen sind auch Unguentum alkoholum lanae aquosum mit Cetiol (Ölsäureoleylester) sowie Unguentum lanette, Cetylstearylalkohol, Cetiol DAB 8, aqua conservata.

Der erfindungsgemäß verwendbaren Zusammensetzung können vorteilhaft auch weitere Vitamine, z.B. Vitamin C, A, $B_1$, $B_2$ und $B_6$ zugesetzt werden.

Die erfindungsgemäßen Salben enthalten als Grundlagen zweckmäßig 70 bis 30 Gew.-%, Wasser vorzugsweise 60 bis 40 Gew.-%,

30 bis 5 Gew.-%, vorzugsweise 25 bis 7 Gew.-%, Cetiol (Oleyloleat),

30 bis 2 Gew.-%, vorzugsweise 25 bis 2 Gew.-% Cetyl-Stearylalkohol oder andere aliphatische Alkohole.

Man kann den Cetyl-Stearylalkohol ganz oder teilweise auch durch andere emulgierende Alkohole ersetzen, z.B. durch aliphatische Alkohole oder Wollwachsalkohol bzw. Diole, Stearinol, mit aliphatischen Säuren veresterte Monoglyceride oder ähnliche Stoffe. Man kann z.B. auch Paraffin oder Vaseline zusetzen, um die Salbe streichfähig zu machen. Auch Cetiol (Oleyloleat) kann durch andere Emulgatoren, z.B. Tween® 20 oder Tween® 80 ganz oder teilweise ersetzt werden. Eine besonders bevorzugte Kombination als Grundlage für Vitamin-E-haltige Salben von Cremes ist jedoch folgende:

30 bis 20 Gew.-% Cetyl-Stearylalkohol,

20 bis 10 Gew.-% Cetiol (oleyl oleat)

60 bis 40 Gew.-% Wasser (aqua conservata).

Es ist bekannt, daß wasserhaltige Salbengrundlagen, wie Unguentum emulsificans aquosum und Unguentum alkoholum lanae aquosum geeignet sind zur Verarbeitung von wasserlöslichen Wirkstoffen. Hier überrascht, daß wasserhaltige Salbengrundlagen, die sogar über 50% Wassergehalt aufweisen können, sehr gut zur Verarbeitung fettlöslicher Wirkstoffe wie Vitamin E geeignet sind.

Überraschenderweise bringen die erfindungsgemäß verwendbaren Zusammensetzungen besondere Vorteile, wenn Vitamin A zugesetzt wird. Insbesondere wird die Behandlungsdauer verkürzt. Demzufolge betrifft die vorliegende Erfindung auch eine Verwendung einer Zusammensetzung zur Herstellung eines Arzneimittels zur Behandlung und zum Schutz der menschlichen und tierischen Haut, die Vitamin A zusammen mit Vitamin E und durchblutungsfördernde Mittel enthalten. Vitamin A kann in Form von Vitamin-A-Palmitat, Vitamin-A-Acetat sowie weiterer Ester des Vitamin A und/oder β-Carotin verwendet werden. Vitamin A soll so ausgewählt sein, daß die maximale Tagesdosis 50.000 I.E. nicht überschreitet, d.h. wenn zwei Darreichungsformen pro Tag angewendet werden, soll die Dosierung bei maximal 25.000 I.E. pro Darreichungsform liegen. Die Vitamin-A-Dosis des erfindungsgemäßen Mittels liegt zwischen 5.000 und 25.000 I.E., vorzugsweise 6.000 bis 15.000 I.E.

Die Vitamine A und E neigen insbesondere in Gegenwart von anderen Wirkstoffen im wäßrigen Medium sehr stark zur Klumpenbildung. Dabei besteht Gefahr, daß die fettlöslichen wertvollen Stoffe nicht absorbiert werden. Überraschend wurde festgestellt, daß geringe Mengen Emulgator, ca. 1%, ausreichen, um die Klumpenbildung zu verhindern. Die Wirkstoffe werden leichter im wässrigen Medium dispergiert bzw. suspendiert. Dies hat den Vorteil, daß die Absorption durch den Darm erleichtert wird. Eine größere Menge Emulgator ist nicht notwendig, da meistens 1 bis 7% ausreichend sind, um die Klumpenbildung zu verhindern. Man kann auch Mengen bis zu 10% oder mehr verwenden. Dabei besteht aber die Gefahr, daß man zuviele Hilfsstoffe zugibt. Die Folge können Nebenwirkungen sein, insbesondere wenn das Medikament längere Zeit verabreicht wird.

Es können die üblichen Emulgatoren, die in den medizinischen Präparaten verwendet werden, wie Tween® 20, Cremophor®, aliphatische Alkohole, partialveresterte Triglyceride. Erfindungsgemäß werden jedoch Tween® 80 und Cetiol bevorzugt. Hierbei wurde beobachtet, daß bei Zugabe von ca. 10% Emulgator die Emulgierung nicht wesentlich besser ist als bei Zusatz von 5% Emulgator.

Man kann als Emulgator auch Lecithin in einer Konzentration zwischen 1 und 13% verwenden. Damit wird die Resorption der Kombination Vitamin A + E, insbesondere aber des Vitamin E begünstigt. Zwar ist auch bei Verwendung von großen Mengen Lecithin bis zu 50% eine positive Wirkung erkennbar. Geringe Mengen des Emulgators aus Lecithin sind aber ausreichend, um die Klumpenbildung der fettlöslichen Vitamine zu verhindern und um eine optimale Resorption zu begünstigen. Es ist ferner zu empfehlen, ca. 1% herkömmliche Emulgatoren wie Tween 80 beizufügen, da sie die Mischbarkeit von Lecithin mit den beiden Vitaminen begünstigen und eine Klumpenbildung verhindern. Besonders vorteilhaft für die Resorption ist die Verwendung von ca. 1% Tween® 80 mit 1 bis 13% Lecithin. Ebenso können die herkömmlichen Emulgatoren Tween® 20, Cetiol (Ölsäureoleylester) und Cremophor® verwendet werden. Als Lecithinpräparat wird das Sojalecithin bevorzugt.

Die erfindungsgemäß zu verwendenden Zusammensetzungen sind auch als Schutz gegen ultraviolettes Licht geeignet. Hierbei werden UV-Stabilisatoren zugesetzt, die hautverträglich sowie fett- und wasserlöslich sind, z.B. Eusolex®. Die UV-Stabilisatoren können in einer Menge von 0,1 bis 20 Gew.-% zugesetzt werden. 0,5 bis 10 Gew.-% werden bevorzugt.

Weitere Zusatzstoffe können Lebertran und/oder ungesättigte Fettsäuren sein, z.B. Linolsäure, Linolensäure oder Ölsäure. Anstelle der ungesättigten Fettsäuren können auch Siliconöle oder Polysiloxane verwendet werden.

Für Hautschutzmittel sind die erfindungsgemäß verwendbaren Zusammensetzungen zur Herstellung eines Arzneimittels zur Behandlung und zum Schutz der menschlichen und tierischen Haut in Kombination mit Phospholipiden z.B. Lecithin geeignet. Durch die Phospholipide wird das Eindringen des Vitamin E in die Haut beschleunigt und dadurch die Wirksamkeit der Vitamin-E-Präparate gesteigert.

Es ist bekannt, daß Bufexamac-Creme oder -salbe zur Behandlung von Hautentzündungen, Allergien, Ekzemen und Juckreizen sich eignet. Überraschenderweise wird jedoch die Behandlungsdauer in Gegenwart von Vitamin E wesentlich verkürzt und die Wahrscheinlichkeit des Rückfalls vermindert. Nach dem Abklingen der Krankheit wird bevorzugt nur mit Vitamin-E-Salbe eingerieben, um einen Rückfall vorzubeugen.

Zur Behandlung von Allergien können die erfindungsgemäß. verwendbaren Zusammensetzungen zur Herstellung eines Arzneimittels zur Behandlung und zum Schutz der menschlichen und tierischen Haut mit antiallergischen Wirkstoffen, insbesondere Antihistaminika kombiniert werden. Der Zusatz von Vitamin E zu solchen antiallergischen Wirkstoffen beschleunigt den Heilungsprozeß.

Als antiallergische Wirkstoffe werden beispielsweise
Clemastinehydrogenfumarat
Chlorphenoxaminehydrochlorid
Dimetidinmaleat
Bamipinlactat oder -hydrochlorid oder andere Salze bzw.
Ester
Propylhexedrinehydrochlorid
Tritoqualine
Dephenhydramin
Meclozinhydrochlorid, verwendet.

Die erfindungsgemäß verwendbaren Zusammensetzungen zur Herstellung eines Arzneimittels zur Behandlung und zum Schutz der menschlichen und tierischen Haut können auch der Förderung des Haarwuchses dienen, insbesondere wenn Aminosäuren zugesetzt werden.

Neben Vitamin E enthalten die verwendbaren Zusammensetzungen zur Herstellung eines Arzneimittels zur Behandlung und zum Schutz der menschlichen und tierischer. Haut die üblichen Träger- und Hilfsstoffe, was insbesondere für die äußerlichen Anwendungen von Bedeutung ist.

Die nachfolgenden Beispiele dienen der näheren Erläuterung der Erfindung:

Beispiel 1

100 g     Salbe enthalten:
400 mg     Allantoin;
400 mg     Dexapanthenol;
5000 mg     D-alpha-Tocopherol;
30000     I.E. Heparin Natrium;

Beispiel 2

100 g     Salbe enthalten:
2,5 g     O-($\beta$-Hydroxyäthyl)-Rutoside;
6,5 g     D-alpha-Tocopherol oder D,L-alpha-Tocopherol;

Beispiel 3

100 g     Salbe enthaltne:
400 mg     Allantoin;
400 mg     Dexapanthenol;

8,8 g      D-alpha-Tocopherol oder D,L-alpha-Tocopherol;
30000     I.E. Heparin Natrium

Beispiel 4

100 g     Salbe enthalten:
4,5 g      Extract Hippocastani (enthält ca. 800 mg Aescin);
5,0 g      D-alpha-Tocopherol;

Beispiel 5

100 g     Gel enthalten:
50000     I.E Heparin Natrium;
12 g       Arnikablüten-Extract ((1;10) Alkohol 60%);
25 g       Tinct. Hippocastani e sem. 1:1 entspricht 0,65 g Aescin;
7,5 g      D-alpha-Tocopherol;

Beispiel 6

100 g     Gel enthalten:
7,0 g      $\beta$-Hydroxyäthyl-Salicylat;
7,0 g      D-alpha-Tocopherol;

Beispiel 7

100 g     Salbe enthalten:
10 g       Benzocain (Anaesthesin);
8 g        D-alpha-Tocopherol-Konzentrat;
1 g        Benzylnicotinat;

Beispiel 8

100 g     Salbe enthalten:
3 g        Hydroxyäthyl-Salicylat;
1 g        Benzylnicotinat;
7 g        D-alpha-Tocopherol;

Beispiel 9

100 g     Salbe enthalten:
8 g        D-alpha-Tocopherol;
400 mg    Allantoin;
400 mg    Dexapanthenol;
150000    I.E. Heparin Natrium;

Beispiel 10

1 Kapsel enthält:
250 mg    Nicotinsäure;
400 mg    D,L-alpha-Tocopherolacetat;
150 mg    Sojabohnenöl;

Beispiel 11

1 Kapsel enthält:
200 mg    $\beta$-Hydroxyäthyl-rutoside;
300 mg    D-alpha-Tocopherolacetat;
180 mg    Sojaöl;

Beispiel 12

1 Kapsel enthält:
150 mg    Extract Hippocastani (enthalten 25 mg Aescin);
300 mg    D-alpha-Tocopherol;
150 mg    Sojaöl;

Beispiel 13

1 Kapsel enthält:
300 mg    Xantinolnicotinat;
400 mg    D-alpha-Tocopherol;
190 mg    Sojaöl;

Beispiel 14

1 Kapsel enthält:
150 mg    Extract Hippocastani (enthalten 25 mg Aescin);
250 mg    Vitamin E;
150 mg    Sojaöl;

Beispiel 15

1 Kapsel enthält:
5 mg      Vitamin $B_1$;
5 mg      Vitamin $B_2$;
5 mg      Vitamin $B_6$;
200 mg    β-Hydoxyäthyl-rutoside;
300 mg    Vitamin E;
50 mg     Nicotinsäureamid;
200 mg    Sojaöl;

Beispiel 16

1 Kapsel enthält:
100 mg    Nicotinsäure;
100 mg    Rosskastanienextract (enthalten 16 mg Aescin);
300 mg    D-alpha-Tocopherolacetat;
200 mg    Sojaöl;

Beispiel 17

1 Kapsel enthält:
200 mg    Inositol Nicotinat;
300 mg    D-alpha-Tocopherol-Konzentrat;
150 mg    Sojaöl;

Beispiel 18

1 Kapsel enthält:
50 mg     Procainhydrochlorid;
400 mg    D-alpha-Tocopherol-Konzentrat;
150 mg    Sojaöl;

Beispiel 19

1 Kapsel enthält:
50 mg     Procainhydrochlorid;

400 mg    D,L-alpha-Tocopherolacetat;
5 mg    Vitamin $B_1$;
5 mg    Vitamin $B_2$;
5 mg    Vitamin $B_6$;
150 mg    Sojaöl oder Maisöl;

Beispiel 20

   Tropfen
100 ml    90% Äthylalkohol enthalten
40 g    D,L-alpha-Tocopherolacetat;
4,5 g    Extract Hippocastani (enthalten 750 mg Aescin);

Beispiel 21

   1 Kapsel enthält:
4,5 mg    entsprechend Dihydroergotoxin-methan-sulphonat;
400 mg    D,L-alpha-Tocopherolacetat;

Beispiel 22

   1 Kapsel enthält:
50 mg    Procain-Hydrochlorid;
200 mg    Nicotinsäure;
400 mg    Vitamin E;
150 mg    Maisöl.

Beispiel 23

   1 Kapsel enthält:
150 mg    Bencyclan-hydrogenfumarat;
400 mg    Vitamin E als D,L-alpha-Tocopherolacetat;
150 mg    Sojaöl.

Beispiel 24

   1 Kapsel enthält:
Pentoxyfyllin      400 mg
Vitamin E      400 mg
Vitamin A Acetat      15.000 I.E.
Sojaöl      120 mg

Beispiel 25

   1 Kapsel enthält:

Naftidirofuryl-Hydrogenoxalat      100 mg

Vitamin E      500 mg

Vitamin A Palmitat      30.000 I.E.

Sojaöl      150 mg

Beispiel 26

   1 Kapsel enthält:

```
Cinnarizin              75 mg
Vitamin E              400 mg
Vitamin A Palmitat     15.000 I.E.
Vitamin B₁, B₂, B₆ zu
gleichen Teilen         10 mg
Vitamin B₁₂              5 µg
Sojaöl                 150 mg
```

Beispiel 27

100 ml Tropfen aus Äthylalkohol enthalten:
Cinnarizin          7,5 g
Vitamin E           4,0 g
Vitamin A Palmitat  2,5 Millionen Einheiten

Beispiel 28

1 Kapsel enthält:

```
Xantinolnicotinat       500 mg
Vitamine E (DL-alpha-
Tocopherol)             400 mg
Vitamin A Palmitat     10.000 I.E.



Tween 80                 20 mg
Sojaöl                  150 mg
```

Beispiel 29

Tropfen in 100 ml Äthylalkohol:

```
Tropfen in 100 ml Äthylalkohol:
Dihydroergotoxinmethansulphonat        1,5 g
aus (0,5 g Dihydroergocristinmethansulphonat)
0,5 g Dihydroergocorninmethansulphonat
0,333 mg alpha-Dihydroergocryptinmethansulphonat
0,167 mg ß-Dihydroergocryptinmethansulphonat)
Vitamin E (DL-alpha-Tocopherolacetat) 3,5 g
Vitamin A Palmitat     2,5 Millionen Einheiten
```

Beispiel 30

1 Kapsel enthält:

```
1 Kapsel enthält:
ß-Pyridyl-carbinol-tartrat                          360 mg
entspricht 150 mg Pyridylcarbinol
D-alpha-Tocopherol                                  400 mg
Vitamin A Palmitat                                  12.000 I.E.
Sojaöl                                              150 mg
```

Beispiel 31

    1 Kapsel enthält:
D,L-alpha-Tocopherol      400 mg
β-Hydroxyäthylrutosid     300 mg
Vitamin A Palmitat        15.000 I.E.
Sojaöl                    150 mg

Beispiel 32

    1 Kapsel enthält:

```
        Ginkoflavonglykoside                        3,0 mg


        Vitamin E (D,L-alpha-Tocopherol-
        acetat)                                     300 mg
        Vitamin A Palmitat                          25.000 I.E.
        Sojaöl                                      100 mg
```

Beispiel 33

    1 Kapsel enthält:
Nicotinsäure          300 mg
Vitamin E             400 mg
Vitamin A Palmitat    15.000 I.E.
Cetiol                20 mg
Sojaöl                150 mg

Beispiel 34

    1 Kapsel enthält:
D,L-alpha-Tocopherolacetat     200 mg
β-Hydroxyäthylrutosid          300 mg
Vitamin A Palmitat             25.000 I.E.
Sojaöl                         120 mg

Beispiel 35

    1 Kapsel enthält:

| Pentoxyfyllin | 400 mg |
| Vitamin E (D,L-alpha-Tocopherol-acetat) | 400 mg |
| Vitamin A Palmitat | 15.000 I.E. |
| Tween ® 80 | 10 mg |
| Sojaöl | 150 mg |

Beispiel 36

1 Kapsel enthält:

| Bamethansulfat | 25 mg |
| D,L-alpha-Tocopherolacetat | 250 mg |
| Vitamin A Palmitat | 10.000 I.E. |
| Sojaöl | 150 mg |

Beispiel 27

1 Kapsel enthält:

| Vincamin | 30 mg |
| Vitamin E (D,L-alpha-Tocopherol-acetat) | 400 mg |
| Vitamin A Palmitat | 30.000 I.E. |
| Sojaöl | 150 mg |

Beispiel 38

| 100 g | Salbe enthalten: |
| 10 g | D-alpha-Tocopherol |
| 50.000 | I.E. Heparin Natrium |
| | die Salbengrundlage besteht aus |
| 22 T | Cetyl-Stearylalkohol |
| 18 T | Cetiol |
| 60 T | Wasser (aqua conservata) |

Beispiel 39

| 100 g | Salbe enthalten: |
| 7 g | Vitamin E (D-alpha-Tocopherol) |
| 1 g | Nicotinsäurebenzylester |
| 1 g | Kampfer |
| | die Salbengrundlage besteht aus |
| 17 T | Cetyl-Stearylalkohol |
| 8 T | Weiße Vaseline |
| 15 T | Cetiol |
| 60 T | Wasser (aqua conservata) |

Beispiel 40

| 100 g | Salbe enthalten: |

11

7 g      Vitamin E
15 g      Tinct. calendulae
           ad 100 g Salbengrundlage aus
13 T      Wollwachsalkohol
2 T      Cetyl-Stearylalkohol
20 T      Cetiol
5 T      Paraffin
50 T      Wasser (aqua conservata)

### Beispiel 41

100 g      Salbe enthalten:
8 g      Vitamin E (D,L-alpha-Tocopherol)
1,5 g      Rosmarinöl
1 g      Extract Hippocastani (standardirisiert auf mind. 8% Aescin)
1 g      Öl juniperi
           Salbengrundlage wie Beispiel 38

### Beispiel 42

     Lösung aus
10 g    Vitamin E (D-alpha-Tocopherol-Konzentrat)
1 g    Latschenkiefernöl (Öl pini pumilionis)
1 g    Eucalyptusöl
1 g    Öl juniperi
1 g    Tween® 80
           ad 100 g Isopropylalkohol

### Beispiel 43

100 g      Salbe enthalten:
7 g      D-alpha-Tocopherol-Konzentrat
2 g      Tinct. arnicae
2 g      Salicyl-$\beta$-Hydroxyäthylester Salbengrundlage wie Beispiel 38

### Beispiel 44

     Lösung gemäß Beispiel 42:
7,0 g      Vitamin E
1,0 g      Latschenkiefernöl
1,0 g      Arnikatinktur
1,0 g      Cetiol
           ad 100 g Isopropylalkohol

### Beispiel 45

100 g      Salbe enthalten:
9,0 g      Vitamin E
20,0 g      Tinct. calendulae
           Salbengrundlage wie Beispiel 38

Beispiele 46 bis 60 betreffen Kombinationen von Vitamin E und A mit Lecithin.

### Beispiel 46

1 Kapsel enthält:

```
Pentoxyfyllin                                       400 mg
Vitamin E (D,L-alpha-Tocopherolacetat) 400 mg
Vitamin-A-Acetat                                    25.000 I.E.
Sojalecithin                                        200 mg
Sojaöl                                              120 mg
Tween Ⓡ 80                                            8 mg
```

Beispiel 47

1 Kapsel enthält:

```
Naftidirofuryl-Hydrogenoxalat           100 mg
Vitamin E (D-alpha-Tocopherol-
Konzentrat)                             500 mg
Vitamin-A-Palmitat                      30.000 I.E.
Sojalecithin                             25 mg
Sojaöl                                  150 mg
```

Beispiel 48

1 Kapsel enthält:

```
Cinnarizin                                      75 mg
Vitamin E (D-alpha Tocopherolacetat)    400 mg
Vitamin-A-Palmitat                      25.000 I.E.
Vitamin B1, B2, B6 zu
gleichen Teilen                          10 mg
Vitamin B12                               5 µg
Sojaöl                                  100 mg
Sojalecithin                            280 mg
```

Beispiel 49

100 ml Tropfen aus Äthylalkohol enthalten

```
Cinnarizin                          7,5 g
Vitamin E                           4,0 g
Vitamin-A-Palmitat     2,5 Millionen Einheiten
Lecithin                            2,5 g
```

Beispiel 50

1 Kapsel enthält:

13

| | |
|---|---|
| Xantinolnicotinat | 500 mg |
| Vitamin E (D,L-alpha-Tocopherol) | 400 mg |
| Vitamin-A-Palmitat | 25.000 I.E. |

| | |
|---|---|
| Tween ® 80 | 20 mg |
| Sojaöl | 150 mg |
| Sojalecithin | 25 mg |

Beispiel 51

| | |
|---|---|
| Tropfen in 100 ml Äthylalkohol | |
| Dihydroergotoxinmethansulphonat | 1,6 g |
| aus | |
| (0,5 g Dihydroergocristinmethansul-phonat | |
| 0,5 g Dihydroergocorninmethansulphonat | |
| 333 mg alpha-Dihydroergocryptinmethan-sulphonat | |
| 167 mg ß-Dihydroergocryptinmethansul-phonat) | |
| Vitamin E (DL-alpha-Tocopherolacetat) | 3,5 g |
| Vitamin-A-Palmitat | 1,5 Millionen Einheiten |
| Sojalecithin | 3,5 g |

Beispiel 52

1 Kapsel enthält:

| | |
|---|---|
| ß-Pyridil-carbinol-tartrat entspricht 150 mg Pyridyl-carbinol | 360 mg |
| D-alpha-Tocopherolacetat | 400 mg |
| Vitamin-A-Palmitat | 10.000 I.E. |
| Sojaöl | 100 mg |
| Sojalecithin | 150 mg |
| Tween ® 20 | 6 mg |

Beispiel 53

1 Kapsel enthält:

| | |
|---|---|
| D,L-alpha-Tocopherol | 400 mg |
| β-Hydroxyäthylrutosid | 300 mg |
| Vitamin-A-Palmitat | 30.000 I.E. |
| Sojaöl | 100 mg |
| Sojalecithin | 250 mg |

Beispiel 54

1 Kapsel enthält:

| | |
|---|---|
| Ginkoflavonglykoside | 3,0 mg |
| Vitamin E (D,L-alpha-Tocopherol-acetat) | 300 mg |
| Vitamin-A-Palmitat | 25.000 I.E. |
| Sojaöl | 100 mg |
| Sojalecithin | 200 mg |

Beispiel 55

1 Kapsel enthält:

| | |
|---|---|
| Nicotinsäure | 300 mg |
| Vitamin E | 400 mg |
| Vitamin-A-Palmitat | 15.000 I.E. |
| Cetiol (Oleylsäureester) | 10 mg |
| Sojaöl | 100 mg |
| Sojalecithin | 20 mg |

Die folgenden Beispiele betreffen Vitamin-E-Präparat enthaltend Dimethylaminoäthanol.

Beispiel 56

1 Kapsel enthält:

| | |
|---|---|
| 20 mg | Dimethylaminoäthanol |
| 400 mg | D,L-alpha-Tocopherolacetat |
| 50 mg | Sojaöl |
| 200 mg | Sojalecithin |
| 200 mg | β-Hydroxyäthylrutosid |

Beispiel 57

1 Kapsel enthält:

| | |
|---|---|
| 20 mg | Dimethylaminoäthanol |
| 400 mg | D,L-alpha-Tocopherolacetat |
| 12.000 | I.E. Vitamin-A-Palmitat (6,67 mg) |
| 100 mg | Sojaöl |
| 300 mg | Lecithin |
| 8 mg | Tween® 80 |
| 75 mg | Cinnarizin |

Beispiel 58

    1 Kapsel enthält:
25 mg    Dimethylaminoäthanolorotat
400 mg    D,L-alpha-Tocopherolacetat
15.000    I.E. Vitamin-A-Palmitat (8,33 mg)
20 mg    Sojalecithin
400 mg    Nicotinsäure

Beispiel 59

    Wie Beispiel 56, jedoch mit 8 mg Tween® 80 angesetzt.

Beispiel 60

    1 Kapsel enthält:
25 mg    Dimethylaminoäthanolorotat
500 mg    D-alpha-Tocopherol-Konzentrat
22.000    I.E. Vitamin-A-Palmitat (12,22 mg)
28 mg    Sojalecithin
120 mg    Sojaöl
3,0 mg    Ginkoflavonglucosid

Beispiel 61

    Kombination gemäß Beispiel 60, jedoch mit 8 mg Tween 20.

Beispiel 62

    1 Kapsel enthält:
30 mg    Dimethylaminoäthanolorotat
400 mg    D,L-alpha-Tocopherolacetat
300 mg    Lecithin
8 mg    Tween® 80
30 mg    Vincamin

Beispiel 63

    1 Kapsel enthält:
25 mg    Dimethylaminoäthanolorotat
350 mg    D-alpha-Tocopherolacetat
15.000    I.E. Vitamin-A-Palmitat
je 5 mg    von Vitamin $B_1$, $B_2$, $B_6$,
5 µg    Vitamin $B_{12}$
15 mg    Nicotinsäureamid
280 mg    Lecithin
75 mg    Cinnarizin

Beispiel 64

    Kombination gemäß Beispiel 63, jedoch mit 5 mg Tween® 80.

Beispiel 65

    1 Kapsel enthält:
25 mg    Dimethylaminoäthanolorotat
400 mg    D,L-alpha-Tocopherolacetat
15.000    I.E. Vitamin-A-Palmitat (8,33 mg)

300 mg    β-Hydroxyäthylrutosid

Beispiel 66

Kombination gemäß Beispiel 65, jedoch mit 8 mg Tween® 80 angesetzt.

Beispiel 67

    1 Kapsel enthält:
35 mg    Dimethylaminoäthanolorotat
500 mg   D-alpha-Tocopherol-Konzentrat
22.000   I.E Vitamin-A-Palmitat (12,22 mg)
400 mg   Xantinolnicotinat

Beispiel 68

    Gemäß Beispiel 67, jedoch mit 4 mg Tween® 20.

Beispiel 69

    1 Kapsel enthält:
30 mg    Dimethylaminoäthanolorotat
400 mg   D,L-alpha-Tocopherolacetat
400 mg   Pentoxyfyllin

Beispiel 70

    1 Kapsel enthält:
35 mg    Dimethylaminoäthanolorotat
350 mg   D-alpha-Tocopherolacetat
15.000   I.E. Vitamin-A-Palmitat
je 5 mg  von Vitamin $B_1$, $B_2$, $B_6$
5 µg     Vitamin $B_{12}$
100 mg   Bencyclanfumarat

Beispiel 71

    Kombination gemäß Beispiel 70, jedoch mit 3 mg Tween® 80.

Beispiel 72

    1 Kapsel enthält:
25 mg    Dimethylaminoethanolorotat
350 mg   D,L-alpha-Tocopherolacetat
17.000   I.E. (9,44 mg) Vitamin-A-Palmitat
70 mg    Sojaöl
75 mg    Cinnarizin

Beispiel 73

    1 Kapsel enthält:
20 mg    Dimethylaminoäthanol
200 mg   D,L-alpha-Tocopherolacetat
12.000   I.E. Vitamin-A-Palmitat (6,67 mg)
50 mg    Sojaöl
250 mg   Sojalecithin

EP 0 343 694 B1

Beispiel 74

1 Kapsel enthält:
35 mg    Dimethylaminoäthanolorotat
400 mg   D,L-alpha-Tocopherolacetat
15.000   I.E. Vitamin-A-Palmitat (8,33 mg)
20 mg    Sojalecithin

Beispiel 75

Kombination gemäß Beispiel 73, jedoch mit 3 mg Tween® 80 angesetzt.

Beispiel 76

1 Kapsel enthält:
20 mg    Dimethylaminoäthanol
200 mg   D,L-alpha-Tocopherolacetat
12.000   I.E. Vitamin-A-Palmitat (6,67 mg)
50 mg    Sojaöl

Beispiel 77

1 Kapsel enthält:
35 mg    Dimethylaminoäthanolorotat
400 mg   D,L-alpha-Tocopherolacetat
15.000   I.E. Vitamin-A-Palmitat (8,33 mg)

Beispiel 78

Kombination gemäß Beispiel 76, jedoch mit 3 mg Tween® 80 angesetzt.

Beispiel 79

1 Kapsel enthält:
35 mg    Dimethylaminoäthanolorotat
500 mg   D-alpha-Tocopherol-Konzentrat
22.000   I.E. Vitamin-A-Palmitat (12,22 mg)

Beispiel 80

Kombination gemäß Beispiel 79, jedoch mit 4 mg Tween® 20.

Beispiel 81

1 Kapsel enthält:
30 mg    Dimethylaminoäthanolorotat
400 mg   D,L-alpha-Tocopherolacetat

Beispiel 82

1 Kapsel enthält:
35 mg    Dimethylaminoäthanolorotat
350 mg   D-alpha-Tocopherolacetat
15.000   I.E. Vitamin-A-Palmitat
je 5 mg  von Vitamin $B_1$, $B_2$, $B_6$
5 µg     Vitamin $B_{12}$
15 mg    Nicotinsäureamid

18

Beispiel 83

Kombination gemäß Beispiel 82, jedoch mit 3 mg Tween® 80.

Beispiel 84

1 Kapsel enthält:
25 mg      Dimethylaminoethanolorotat
350 mg     D,L-alpha-Tocopherolacetat
17.000     I.E. (9,44 mg) Vitamin-A-Palmitat
70 mg      Sojaöl
In allen Beispielen wurde Sojaöl zwischen 50 und 200 mg pro Kapsel zugesetzt. Es können auch andere neutrale Öle wie Olivenöl, Rüböl etc. verwendet werden.

Beispiel 85

Lösung zur äußerlichen Anwendung, insbesondere in Form von Spray aus:
33 T   D-alpha-Tocopherolkonzentrat
8 T    Phosphorlipide
99 T   Isopropylalkohol
45 T   Isopropylalkohol (70%)

Beispiel 86

1 Kapsel enthält:
30 mg      Chlorphenoxaminehydrochlorid
400 mg     D,L-alpha-Tocopherolacetat
50 mg      Nicotinsäure
100 mg     Sojaöl

Beispiel 87

Gemäß Beispiel 86, jedoch anstelle von 30 mg Chlorphenoxaminehydrochlorid werden 40 mg verwendet und 20.000 I.E. Vitamin-A-Palmitat.

Beispiel 88

1 Kapsel enthält:
70 mg      Bamipinhydrochlorid
500 mg     D-alpha-Tocopherolkonzentrat
100 mg     Troxerutin
80 mg      Erdnußöl

Beispiel 89

1 Kapsel enthält:
120 mg     Tritoqualine
300 mg     D-alpha-Tocopherolacetat
100 mg     Troxerutin
80 mg      Sojaöl

Beispiel 90

Eine Salbe enthält:
40,0 g       Clemastinehydrogenfumarat
8,0 g        D-alpha-Tocopherolkonzentrat
60.000       I.E. Heparin Natrium
ad 100,0 g   eine Salbe aus 20,0 g Cetiol (Ölsäureoleylester)

20,0 g        Cetylstearylalkohol
60,0          aqua Conservata

Beispiel 91

Eine Salbe enthält:
1,5 g         Chlorphenoxaminhydrochlorid
10 g          D,L-alpha-Tocopherol
50.000        I.E. Heparin Natrium
ad 100,0 g    Salbe wie Beispiel 90

Beispiel 92

1 Kapsel enthält:
70 mg         Bamipinhydrochlorid
400 mg        D-alpha-Tocopherolkonzentrat
30 mg         Cinnarizin

Beispiel 93

Gemäß Beispiel 90, jedoch mit 3% Calendulaeöl.

Beispiel 94

Eine Salbe enthält:
5 g           D-alpha-Tocopherolkonzentrat
10.000        I.E. Vitamin-A-Palmitat
50.000        I.E. Heparin Natrium
ad 100,0 g    Eucerinanhydricum

Beispiel 95

Das gleiche wie Beispiel 94, jedoch mit 8 g D-alpha-Tocopherolkonzentrat.
Bei den folgenden Kombinationen handelt es sich um Salben enthaltend Bufexamac zur Behandlung von Hautentzündungen.
Die folgenden Beispiele betreffen Lichtschutzmittel.

Beispiel 96

Eine Salbe enthält:
8 g           D-alpha-Tocopherolkonzentrat
2 g           Phospholipide
2 g           4-Phenylbenzophenon-2-Carbonsäure-Isooctylester
ad 100 g      Salbengrundlage aus
22 Teile      Cetyl-Stearylalkohol
18 Teile      Cetiol Ölsäureoleylester
60 Teile      Wasser

Beispiel 97

100 g         Salbe enthalten:
3 g           Hydroxyäthylsalicylat
1 g           Benzylnicotinat
8 g           D-alpha-Tocopherolkonzentrat
2 g           Phospholipide
ad 100,0 g    Salbengrundlage wie Beispiel 96

Beispiel 98

| | |
|---|---|
| 100 g | Salbe enthalten: |
| 50.000 | I.E. Heparin Natrium |
| 12,0 g | Arnica Lebutenextract 1 = 10 (Alkal 60%) |
| 7,5 g | D-alpha-Tocopherolkonzentrat |
| 3 g | Phospholipide |
| 0,5 g | Octadecadeinsäure |
| 0,15 g | Linolsäure |
| ad 100,0 g | Salbengrundlage wie 96 |

Beispiel 99

| | |
|---|---|
| | Eine Salbe enthält: |
| 10,0 g | D-alpha-Tocopherol-Konzentrat |
| 2,0 g | Benzaron |
| 2 g | Phospholipide |
| ad 100,0 g | Salbengrundlage wie 96 |

Beispiel 100

| | |
|---|---|
| | 1 Kapsel enthält: |
| 35 mg | Xanthaxanthin |
| 8 mg | β-Carotin |
| 8 mg | Calcium D Pantothenat |
| 0,15 mg | D-Biotin |
| 250 mg | Lecithin |
| 400 mg | D,L-alpha-Tocopherolacetat |
| 80 mg | Sojabohnenöl |

Beispiel 101

| | |
|---|---|
| | 1 Kapsel enthält: |
| 30 mg | Xanthaxanthin |
| 300 mg | D-alpha-Tocopherolkonzentrat |
| 150 mg | Sojabohnenöl |

Beispiel 102 bis 107 und Vergleichsbeispiel

Acht Lichtschutzmittel zur Bestimmung der erythemverhütenden Wirkung wurden am Menschen getestet. Dabei handelte es sich um die folgenden:

Beispiel 102

"DL Hep Na"
mit 8 Gew.-% D,L-alpha-Tocopherol und
50. 000 Einheiten Heparin Natrium

Beispiel 103

"Vitamin E COO/8
55.000 Hepa Na"
mit 8 Gew.-% natürlichem Vitamin E und 55.000 I.E. Heparin Natrium

Beispiel 104

"Vitamin E + COO
BN 8/1.5"

mit 8 Gew.-% natürlichem Vitamin E und 1,5 Gew.-% Benzylnicotinat

Beispiel 105

"8 Gew.-% Covitol und
4 Gew.-% Arnika"
mit 8 Gew.-% natürlichem Vitamin E und 2 Gew.-% Arnikaöl

Beispiel 106

"8 Gew.-% Covitol
2 Gew.-% Campher
2 Gew.-% Menthol
10 Gew.-% Pfefferminzöl
80 Gew.-% Lanette"
mit 8 Gew.-% natürlichem Vitamin E und den vorher bezeichneten Nebeninhaltsstoffen.

Beispiel 107

"8 Gew.-% Covitol
10 Gew.-% Calendulaeöl
82 Gew.-% Lanette"
mit 8 Gew.-% natürlichem Vitamin E.

Vergleichsbeispiel

"Unguentum Lanette DHW Art. 226/15270"

Bei allen Produkten mit Ausnahme der Lanette-Salbe, handelte es sich um weiße, salbige Emulsionen. Die Produkte erfüllten die üblichen, an solche Kosmetika zu stellenden Anforderungen. Sie ließen sich leicht und gleichmäßig auf die Haut auftragen und erzeugten hier keine Schicht, die durch übermäßigen Glanz, Klebrigkeit oder Fettigkeit aufgefallen wäre.

Bei der Anwendung gab keine der Versuchspersonen eine Mißempfindung wie Jucken, Brennen oder Spannen der Haut oder übermäßiges Kälte- oder Wärmegefühl an.

Bei den Untersuchungen der Haut nach 24 Stunden zeigten sich außerhalb der Erythemreaktionen keine Hautveränderungen, die für eine (photo) toxische oder (photo)allergische Wirkung der Produkte gesprochen hätten.

Untersuchungsmethode:

Die Bestimmung der Lichtschutzwirkung erfolgte als mittlerer Schutzfaktor nach SCHULZE an 20 hautgesunden Versuchspersonen unterschiedlichen Geschlechts, Alters und Hauttyps, entsprechend der DIN-Norm 67 501, wenn nicht ausdrücklich etwas anderes vermerkt wird. Die Anwendung der Produkte erfolgte einheitlich in einer Schichtdicke von 150 mg/100 cm².

Zur Lichtexposition wurden wie üblich vier Osram[R]-Ultravitalux-Lampen im Abstand von 30 cm zueinander und zum Rücken des Probanden benutzt. Die Erythemschwellenzeit wurde nach etwa 24 Stunden abgelesen. Der individuelle Schutzfaktor stellt den Quotienten aus der Erythemschwellenzeit der geschützten Haut und der Erythemschwellenzeit der ungeschützten Haut dar. Etwaige Zwischenwerte (z.B. 1.7, 3.4 usw.) ergeben sich dann, wenn die beiden dem Testfeld benachbarten Kontrollfelder unterschiedliche Erythemschwellenzeiten aufweisen oder das Testfeld eine geringere Intensitätszunahme erkennen läßt als die Kontrollfelder.

Ergebnisse:

Bei der so an 20 Probanden unterschiedlichen Alters, Geschlechts und Hauttyps vorgenommenen Prüfung wurden die folgenden mittleren Schutzfaktoren (unterstrichen) als arithmetische Mittel aus den angegebenen Einzelwerten (in Klammern) erhalten:

Beispiel 102

$\underline{4.38}$ (1x2.0, 2x2.8, 11x4.0, 5x5.6, 1x8.0)

Beispiel 103

$\underline{4.02}$ (2x2.0, 9x2.8, 6x4.0, 2x8.0, 1x11.2)

Beispiel 104

$\underline{4.21}$ (1x1.4, 5x2.0, 4x2.8, 3x4.0, 4x5.6, 2x8.0, 1x11.2)

Beispiel 105

$\underline{2.72}$ (4x1.4, 3x2.0, 9x2.8, 3x4.0, 1x5.6)

Beispiel 106

$\underline{2.88}$ (6x2.0, 10x2.8, 3x4.0, 1x5.6)

Beispiel 107

$\underline{3.94}$ (2x2.0, 5x2.8, 9x4.0, 3x5.6, 1x8.0)

Vergleichsbeispiel

Lanette-Salbe
$\underline{1.07}$ (1x0.7, 15x1.0, 4x1.4)
Ein handelsübliches Präparat (Standardpräparat K 17 N), das bisher an 220 Probanden einen mittleren Schutzfaktor von 3.82 gezeigt hatte, wies bei dieser Prüfung einen arithmetischen Mittelwert von 3.74 auf.

Aufgrund der obigen Werte und bei deren Vergleich mit den an zahlreichen Versuchs- und Handelsprodukten unter den gleichen Bedingungen und in der Praxis erhaltenen Egebnissen sind die Produkte 105 und 106 als knapp mittel-wirksame Lichtschutzmittel, die übrigen Produkte dagegen als mittel- bis stark-wirksame Lichtschutzmittel zu bezeichnen.

Während die Grundlage, das Unguentum-Lanette, erwartungsgemäß keinerlei Lichtschutzwirkung aufwies, lassen sich die übrigen sechs Produkte aufgrund teilweise statistisch signifikanter Unterschiede in zwei Gruppen einteilen. Die eine, verhältnismäßig schwach-wirksame Gruppe, enthält neben 8 Gew.-% Vitamin E entweder wie das Produkt 106 verschiedene Zusätze oder wie das Produkt 105 Arnikaöl.

Dem ist eine sehr viel stärker wirksame Gruppe gegenüberzustellen, die entweder wie die "Produkte 102 und 103" Heparin enthalten oder aber wie das "Produkt 104" 1,5 Gew.-% Benzylnicotinat oder das "Produkt 107" 10 Gew.-% Calendulaeöl.

Diese Unterschiede berechtigen zu mehreren Schlüssen. Zunächst ist festzustellen, daß Vitamin E sowohl in natürlicher wie auch in racemischer Form eine deutliche Lichtschutzwirkung besitzt, die offenbar, vor allem durch Zusatz von Heparin, erhöht werden kann. Ähnlich wirksam ist auch ein verhältnismäßig hoher Zusatz an Calendulaeöl sowie überraschenderweise auch das hyperaemesierende Benzylnicotinat.

## Patentansprüche

1.  Verwendung einer Zusammensetzung, die neben üblichen Träger- und/oder Hilfsstoffen als wesentlichen Bestandteil Vitamin E als alpha-Tocopherol und/oder dessen Ester natürlicher oder synthetischer Herkunft in Mengen von 200 bis 1000 mg pro Darreichungseinheit sowie durchblutungsfördernde und/oder gefäßerweiternde Mittel und außerdem gegebenenfalls Vitamin C, Vitamin A, Vitamine der B-Reihe, Phospholipide, ungesättigte Fettsäuren und/oder Emulgatoren enthält, zur Herstellung eines Arzneimittels in Kapselform zur Behandlung und zum Schutz der menschlichen und tierischen Haut.

2.  Verwendung einer Zusammensetzung, die neben üblichen Träger- und/oder Hilfsstoffen als wesentlichen Bestandteil Vitamin E als alpha-Tocopherol und/oder dessen Ester natürlicher oder synthetischer Herkunft in Mengen von 0,5 bis 20 Gew.-% pro Darreichungseinheit sowie durchblutungsfördernde und/oder gefäßerweiternde Mittel und außerdem gegebenenfalls Vitamin C, Vitamin A, Vitamine der B-Reihe, Phospholipide, ungesättigte Fettsäuren und/oder Emulgatoren enthält, zur Herstellung eines Arzneimittels in Form von Crème, Gel, Salbe, Milch, Lotion oder Lösung zur Behandlung und zum Schutz der menschlichen und tierischen Haut.

3. Verwendung einer Zusammensetzung, die neben üblichen Träger- und/oder Hilfsstoffen als wesentlichen Bestandteil Vitamin E als alpha-Tocopherol und/oder dessen Ester natürlicher oder synthetischer Herkunft in Mengen bis zu 32 Gew.-% pro Darreichungseinheit sowie durchblutungsfördernde und/oder gefäßerweiternde Mittel, und außerdem gegebenenfalls Vitamin C, Vitamin A, Vitamine der B-Reihe, Phospholipide, ungesättigte Fettsäuren und/oder Emulgatoren enthält, zur Herstellung eines Arzneimittels in Form von Spray, Tinktur oder Lösung in Alkohol zur Behandlung und zum Schutz der menschlichen und tierischen Haut.

## Claims

1. Use, for the preparation of a medicament in the form of capsules for the treatment and protection of human and animal skin, of a composition containing, as the essential constituent in addition to conventional carriers and/or excipients, vitamin E as alpha-tocopherol and/or its esters of natural or synthetic origin in amounts of from 200 to 1,000 mg per dosage unit, as well as blood circulation-promoting agents and/or vasodilators, and in addition optionally vitamin C, vitamin A, vitamins of the B-series, phospholipids, unsaturated fatty acids and/or emulsifiers.

2. Use, for the preparation of a medicament in the form of a cream, gel, ointment, milk, lotion or solution for the treatment and protection of human and animal skin, of a composition containing, as the essential constituent in addition to conventional carriers and/or excipients, vitamin E as alpha-tocopherol and/or its esters of natural or synthetic origin in amounts of from 0.5 to 20% by weight per dosage unit, as well as blood circulation-promoting agents and/or vasodilators, and in addition optionally vitamin C, vitamin A, vitamins of the B-series, phospholipids, unsaturated fatty acids and/or emulsifiers.

3. Use, for the preparation of a medicament in the form of a spray, tincture or solution in alcohol for the treatment and protection of human and animal skin, of a composition containing, as the essential constituent in addition to conventional carriers and/or excipients, vitamin E as alpha-tocopherol and/or its esters of natural or synthetic origin in amounts of up to 32% by weight per dosage unit, as well as blood circulation-promoting agents and/or vasodilators, and in addition optionally vitamin C, vitamin A, vitamins of the B-series, phospholipids, unsaturated fatty acids and/or emulsifiers.

## Revendications

1. Utilisation d'une composition, qui, en dehors de supports et/ou d'adjuvants usuels, contient, comme constituant principal, de la vitamine E sous la forme d'alpha-tocophérol et/ou ses esters d'origine naturelle ou synthétique en des quantités allant de 200 à 1000 mg par dose unitaire, ainsi que des agents favorisant l'irrigation sanguine et/ou vasodilatateurs et en outre, éventuellement, de la vitamine C, de la vitamine A, des vitamines du groupe B, des phospholipides, des acides gras insaturés et/ou des émulsifiants, pour la fabrication d'un médicament sous la forme de capsules pour le traitement et la protection de la peau humaine et de la peau animale.

2. Utilisation d'une composition qui, en dehors de supports et/ou d'adjuvants usuels, contient, comme constituant principal, de la vitamine E sous la forme d'alpha-tocophérol et/ou ses esters d'origine naturelle ou synthétique en des quantités allant de 0,5 à 20 % en poids par dose unitaire, ainsi que des agents favorisant l'irrigation sanguine et/ou vasodilatateurs et en outre, éventuellement, de la vitamine C, de la vitamine A, des vitamines du groupe B, des phospholipides, des acides gras insaturés et/ou des émulsifiants, pour la fabrication d'un médicament sous la forme d'une crème, d'un gel, d'une pommade, d'un lait, d'une lotion et d'une solution pour le traitement et la protection de la peau humaine et de la peau animale.

3. Utilisation d'une composition qui, en dehors de supports et/ou d'adjuvants usuels, contient, comme constituant principal, de la vitamine E sous la forme d'alpha-tocophérol et/ou ses esters d'origine naturelle ou synthétique en des quantités atteignant jusqu'à 32 % en poids par dose unitaire, ainsi que des agents favorisant l'irrigation sanguine et/ou vasodilatateurs et en outre, éventuellement, de la vitamine C, de la vitamine A, des vitamines du groupe B, des phospholipides, des acides gras insaturés et/ou des émulsifiants, pour la fabrication d'un médicament sous la forme d'un produit à pulvériser, d'une teinture ou d'une solution dans de l'alcool pour le traitement et la protection de la peau humaine et de la peau animale.